# EUROPEAN PATENT APPLICATION

(11) **EP 3 865 138 A1**
(43) Date of publication of application: **18.08.2021**
(21) Application number: 19870358.9
(22) Date of filing: 05.09.2019
(51) Int. Cl.: A61K 31/716, A23K 10/16, A23K 20/163, A23K 50/80, A23L 33/145, A61K 31/215, A61K 31/683, A61K 36/064, A61P 31/00, A61P 37/04

(54) **IMMUNOSTIMULATOR AND METHOD FOR PREVENTING INFECTION**

(30) Priority: 09.10.2018 JP 2018191154
(71) Applicant: Asahi Group Holdings, Ltd., Tokyo 130-8602 (JP)
(72) Inventor: SHIRAI, Takeshi, Moriya-shi, Ibaraki 302-0106 (JP); YOSHIDA, Jumpei, Moriya-shi, Ibaraki 302-0106 (JP)
(74) Representative: Müller-Boré & Partner Patentanwälte PartG mbB
(86) International application number: PCT/JP2019/034989
(87) International publication number: WO 2020/075424

(57) **Abstract**

Provided is an immunostimulator containing glucan and having an immunostimulatory effect. This immunostimulator contains glucan and lipid derived from yeast cell wall and is water-insoluble. A total content percentage of glucan and lipid is 80 mass% or more, and a content ratio of lipid to glucan is 0.1 or more and 0.4 or less.

## Description

### TECHNICAL FIELD

The present invention relates to an immunostimulator and a method for preventing infection.

### BACKGROUND ART

In recent years, there are concerns about the worldwide spread of drug-resistant bacteria. The World Health Organization (WHO) has pointed out an abuse of livestock antibiotics as one of the causes, and the livestock industry is calling for alternatives to antibiotics, i.e., solutions to infectious diseases. Improvement of an immune function inherent in a living body is effective as a countermeasure against infectious diseases, and expectations are placed on β-glucan, which is widely recognized as a material having an immunostimulatory effect.

In this regard, Japanese Unexamined Patent Application Publication (Translation of PCT Application) No. 2017-511122 describes a method for treating a yeast cell wall to obtain β-glucan soluble in dimethyl sulfoxide. Japanese Laid-Open Patent Publication No. 2009-263333 describes a composition containing selenium-enriched yeast and β-glucan and considered as having an immunostimulatory effect. Japanese Laid-Open Patent Publication No. 2004-099580 describes an immunoenhancing composition prepared by autolyzing yeast cells. Japanese Laid-Open Patent Publication No. 2003-169607 describes a fish and shellfish culturing feed to which a yeast decomposition composition obtained by autolyzing yeast is added and which is considered as improving immune activity.

### SUMMARY OF THE INVENTION

### PROBLEM TO BE SOLVED BY THE INVENTION

Although a number of materials containing β-glucan are distributed in the market, the materials have problems such as large variation in quality depending on a product and the presence of many products without guarantee of functionality. Therefore, an object of an aspect of the present invention is to provide an immunostimulator containing glucan and having an immunostimulatory effect.

### MEANS FOR SOLVING PROBLEM

A first aspect of the present invention provides an immunostimulator comprising glucan and lipid derived from a yeast cell wall, the immunostimulator having a total content percentage of glucan and lipid of 80 mass% or more, having a content ratio of lipid to glucan of 0.1 or more and 0.4 or less, and being water-insoluble. In one embodiment, a content ratio of α-glucan to β-glucan may be 0.2 or more and 0.85 or less. In one embodiment, the immunostimulator may comprise mannan, and a content percentage of mannan may be 5 mass% or less. In one embodiment, the immunostimulator may be used for fish.

A second aspect provides a food/drink or a feed comprising the immunostimulator. A third aspect provides an infection preventive agent for fish comprising glucan and lipid, the infection preventive agent having a total content percentage of glucan and lipid of 80 mass% or more. A fourth aspect provides a method for preventing infection comprising administering to a subject the immunostimulator.

A fifth aspect provides a method for manufacturing yeast-derived glucan, comprising: preparing a composition containing a yeast cell wall subjected to an autolysis treatment; subjecting the composition containing the yeast cell wall to an alkaline hydrolysis treatment to obtain a hydrolysate; and recovering glucan from the hydrolysate.

In one embodiment, the composition containing the yeast cell wall is obtained by a method including subjecting a yeast to an autolysis treatment to obtain an autolysate, and subjecting the autolysate to a centrifuging treatment. In one embodiment, the alkali hydrolysis treatment is performed by heating in the presence of an alkali metal hydroxide. In one embodiment, the yeast-derived glucan has a content percentage of mannan of 5 mass% or less. In one embodiment, the yeast-derived glucan has a content percentage of protein of 10 mass% or less. In one embodiment, the yeast-derived glucan has a coefficient of variation of glucan content percentage of 10 % or less.

### EFFECT OF THE INVENTION

According to an aspect of the present invention, the immunostimulator containing glucan and having an immunostimulatory effect can be provided.

### BRIEF DESCRIPTION OF DRAWINGS

Fig. 1 is a graph showing an ability to promote production of reactive oxygen species for porcine leukocytes.
Fig. 2 is a graph showing an ability to promote production of reactive oxygen species for human neutrophil-like cells.
Fig. 3 is a graph showing a survival rate of a vibriosis infection test for rainbow trout.

### MODES FOR CARRYING OUT THE INVENTION

The term "step" as used herein includes not only an independent step but also a step not clearly distinguishable from another step as long as the intended purpose of the step is achieved. If multiple substances correspond to a component in a composition, the content of the component in the composition means the total amount of the multiple substances present in the composition unless otherwise specified. The content of each component is a dry matter-equivalent value of an immunostimulator and may be an average value. An average value is used as the content in this case in consideration of variations between production lots and is, for example, an arithmetic mean value for 6 or more arbitrarily selected samples. An upper limit of the number of samples for calculating the average value is 20 or less, for example. Embodiments of the present invention will now be described in detail. However, the embodiments described below exemplify an immunostimulator etc. for embodying the technical idea of the present invention, and the present invention is not limited to the immunostimulator etc. described below.

### Immunostimulator

The immunostimulator contains glucan and lipid, and the total content percentage of glucan and lipid is 80 mass% or more. The immunostimulator according to the present embodiment has good quality stability and a clear immunostimulatory effect. This not only is expected to suppress the onset and progression of infectious diseases in livestock, humans, cultured fish, etc., but also may provide a means for reducing a threat of development of drug-resistant bacteria. The glucan and lipid constituting the immunostimulator are preferably derived from a yeast cell wall, for example, and are preferably obtained by a hydrolysis pretreatment of the yeast cell wall. The hydrolysis treatment includes alkaline hydrolysis and acid hydrolysis.

The glucan constituting the immunostimulator is a polymer in which D-glucose is linked by glycosidic bonds and includes β-glucan and α-glucan. For example, β-glucan has a linear main chain skeleton of β-1,3 linkage and a side chain branched by β-1,6 linkage. For example, α-glucan has a linear main chain skeleton of α-1,4 linkage and a side chain branched by α-1,6 linkage.

The total content percentage of glucan in the immunostimulator is, for example, 60 mass% or more, preferably 65 mass% or more, more preferably 70 mass% or more and, for example, 90 mass% or less, preferably 85 mass% or less, more preferably 80 mass % or less.

The content percentage of β-glucan in the immunostimulator is, for example, 30 mass% or more, preferably 35 mass% or more, or 40 mass% or more and, for example, 65 mass% or less, preferably 60 mass% or less, or 50 mass% or less. The content percentage of α-glucan is, for example, 10 mass% or more, preferably 15 mass% or more, or 20 mass% or more and, for example, 40 mass% or less, preferably 35 mass% or less, or 30 mass% or less.

The ratio of the α-glucan content to the β-glucan content in the immunostimulator is, for example, 0.2 or more and 0.85 or less, preferably 0.25 or more, 0.3 or more, 0.4 or more, 0.45 or more, or 0.5 or more, and preferably 0.8 or less, 0.7 or less, or 0.6 or less. In a case where the content ratio of α-glucan to β-glucan is within the range, the immunostimulating activity tends to be further enhanced.

The lipid in the immunostimulator includes simple lipid formed by ester bonding of alcohol and fatty acid, complex lipid that is lipid containing phosphate, sugar, protein, etc. in molecules, and derived lipid derived from the simple lipid or the complex lipid by hydrolysis. The total content percentage of lipid in the immunostimulator is, for example, 3 mass% or more, preferably 5 mass% or more, or 10 mass% or more and, for example, 20 mass% or less, preferably 16 mass% or less, or 14 mass% or less. In a case where the content percentage of lipid is within the range, the immunostimulating activity tends to be further enhanced.

The total content percentage of glucan and lipid in the immunostimulator is 80 mass% or more, preferably 82 mass% or more. The total content percentage is, for example, 95 mass% or less, preferably 90 mass% or less, or 88 mass% or less. In a case where the total content percentage of glucan and lipid is within the range, the immunostimulating activity tends to be further enhanced.

The ratio of the total content of lipid to the total content of glucan in the immunostimulator is, for example, 0.1 or more and 0.4 or less, preferably 0.12 or more, or 0.15 or more and, for example, 0.3 or less, preferably 0.25 or less, or 0.2 or less. In a case where the content ratio of lipid to glucan is within the range, the immunostimulating activity tends to be further enhanced.

The immunostimulator may further contain mannan in addition to the glucans and lipids. Mannan is a polysaccharide having D-mannose as a main constituent unit. In a case where the immunostimulator contains mannan, the content percentage of mannan is, for example, 5 mass% or less, preferably 3 mass% or less, or 1.2 mass% or less and, for example, 0.1 mass% or more, preferably 0.2 mass% or more, or 0.3 mass% or more. In a case where the content percentage of mannan is within the range, the immunostimulating activity tends to be further enhanced. The ratio of the total content of mannan to the total content of glucan in the immunostimulator is, for example, 0.004 or more and 0.05 or less, preferably 0.005 or more, or 0.008 or more, and preferably 0.02 or less, or 0.018 or less.

The immunostimulator may further contain a protein in addition to the glucan and lipid. In a case where the immunostimulator contains protein, the content percentage of protein is, for example, 10 mass% or less, preferably 8 mass% or less, or 6 mass% or less and, for example, 1 mass% or more, preferably 2 mass% or more, or 3 mass% or more. In a case where the content percentage of protein is within the range, the immunostimulating activity tends to be further enhanced.

The ratio of the total content of mannan and protein to the total content of glucan in the immunostimulator is, for example, 0.01 or more and 0.2 or less, preferably 0.02 or more, or 0.05 or more, and preferably 0.1 or less, or 0.09 or less.

The immunostimulator may further contain ash. In a case where the immunostimulator contains ash, the content percentage of ash is, for example, 10 mass% or less, preferably 6 mass% or less, or 4 mass% or less and, for example, 1 mass% or more, preferably 1.5 mass% or more, or 2 mass% or more. In a case where the content percentage of ash is within the range, the immunostimulating activity tends to be further enhanced.

The contents of the components of the immunostimulator can be measured by a conventional method. For example, the content of glucan can be measured by quantifying glucose generated by hydrolyzing glucan. The content of lipid can be quantified by an acid decomposition method.

The immunostimulator has excellent quality stability. The stability of the quality of the immunostimulator can be evaluated by variations in content percentage of each component due to a difference in production lot, for example. Specifically, the stability is evaluated by a coefficient of variation (%) of the content percentage of each component. The coefficient of variation (%) is calculated a percentage of a value obtained by measuring the content percentage of each component of the immunostimulator for 6 or more arbitrarily selected samples, calculating an arithmetic mean value and a standard deviation of the content percentage, and dividing the standard deviation by the arithmetic mean value. The coefficient of variation (%) of the total content percentage of glucan in the immunostimulator is, for example, 10 % or less, preferably 8 % or less, or less than 5 %. The coefficient of variation (%) of the content percentage of lipid is, for example, 20 % or less, preferably 15 % or less, or 8 % or less. The coefficient of variation (%) of the total content percentage of glucan and lipid is, for example, 6 % or less, preferably 5 % or less, or less than 5 %. The lower limit of the coefficient of variation is 0 % and is actually a value larger than 0 %.

The immunostimulator may further contain a known component having an immunoregulatory effect in addition to the main components, i.e., glucan and lipid. Examples of the component having an immunoregulatory effect include tea extract, fucoidan, arabinoxylan, lactoferrin, catechin, chitosan, chitosan oligosaccharide, chitin oligosaccharide, L-ascorbic acid, coenzyme Q₁₀ (including a reduced form), etc.

The immunostimulator can contain any component known as a component that may usually be used in pharmaceuticals, foods, feeds, etc. as needed, such as water, fat and oil, sugars, vitamins, sweeteners, seasonings, acidulants, preservatives, flavors, colorants, excipients, expanders, binders, thickeners, stabilizers, emulsifiers, and pH adjusters.

A form of the immunostimulator may be any of solids such as powders and granules, liquids, pastes, etc. A dosage form of the immunostimulator can appropriately be selected depending on an administration method, a subject of administration, etc. Examples of the dosage form include formulations for oral administration such as tablets, capsules, granules, troches, powders, and liquids. These formulations can be manufactured in accordance with a known method by using additives such as excipients, lubricants, binders, disintegrants, stabilizers, flavoring agents, and diluents. The immunostimulator may be contained to make up a food composition such as a health food or a dietary supplement, a beverage composition, a feed, etc.

When administered to a subject of administration such as a vertebrate, the immunostimulator causes an immunostimulating action to occur in the subject. As a result, an onset of infectious diseases in the subject can be suppressed. Therefore, the immunostimulator can be applied to a method for preventing infectious diseases in the subject. Examples of vertebrates receiving the administration include mammals, birds, fishes, etc. Mammals may include humans and may be non-human mammals. Examples of non-human mammals include pigs, cattle, horses, sheep, monkeys, and pet animals such as dogs and cats. Examples of birds include meat chickens, laying hens, turkeys, and ducks, pigeons. Examples of fishes include salmonids such as salmon, trout, yamame trout, char, and Ito, carp, crusian carp, tilapia, catfish, Japanese sea bass, Japanese amberjack, greater amberjack, yellowtail, flatfish, sea bream, tuna, and eel.

When the immunostimulator is administered as a pharmaceutical agent or food/drink, for example, an amount of 1 mg to 500 mg (dry weight)/kg body weight can orally be administered once to several times a day. When the immunostimulator is used as food, drink, feed, etc., 0.1 g to 1 g can be added per 100 g of food, drink, feed, etc.

When administered as feed, for example, for mammals, birds, and fishes, the immunostimulator can be mixed to 0.001 mass% or more and 1 mass% or less with the feed to be administered and can be administered once to several times a day.

### Method for Manufacturing Immunostimulator

The immunostimulator can be manufactured by the following manufacturing method using a yeast cell wall as a raw material, for example. The method for manufacturing the immunostimulator includes, for example, a hydrolysis step of hydrolyzing the yeast cell wall with an acid aqueous solution or an alkaline aqueous solution to obtain a hydrolysate, and a recovery step of recovering a glucan-containing composition from the hydrolysate. Therefore, the immunostimulator may be a composition containing yeast-derived glucan.

The yeast cell wall subjected to the hydrolysis step may be pretreated in a pretreatment step. The pretreatment step includes an autolysis step of autolyzing yeast used as a raw material to obtain an autolysate, a hot water extraction step of subjecting the yeast used as a raw material to hot water extraction to obtain a hot water extract, a pre-decomposition step such as an enzyme treatment step of treating the yeast used as a raw material with an enzyme to obtain an enzyme-treated product, and a first centrifugation step of centrifuging a pre-decomposition product such as an autolysate, a hot water extract, and an enzyme-treated product to obtain the yeast cell wall.

Examples of the yeast used as a raw material include yeasts of Saccharomyces, Schizosaccharomyces, Kluyberomyces, Candida, Pichia, and Torulopsis, at least one selected from the group consisting of these genera is preferable, and yeasts of Saccharomyces is more preferable. The yeasts of Saccharomyces include, for example, Saccharomyces cerevisiae, Saccharomyces pastorianus, Saccharomyces bayanus, etc., and may be at least one selected from the group consisting of these yeasts. The yeasts are also referred to as beer yeast, whiskey yeast, shochu yeast, baker's yeast, wine yeast, sake yeast, bioethanol yeast, etc. depending on its use, and may be at least one selected from the group consisting of these yeasts. One yeast may be used alone or two or more yeasts may be used in combination as a raw material.

In the autolysis step, protein etc. of yeast are decomposed by protease of the yeast itself to obtain an autolysate. For example, the autolysis step is performed by adjusting pH from 2 to 7 and temperature to 40 °C or higher and 65 °C or lower for 1 hour or more and 48 hours or less. In the first centrifugation step, the autolysate is centrifuged to obtain a fraction containing yeast cell walls as a heavy liquid. For centrifugation, for example, a nozzle centrifuge, an intermittent discharging centrifuge, a Sharples centrifuge, etc. can be used in industrial production, and conditions for centrifugation can appropriately be adjusted as long as an insoluble component in the autolysate can be recovered.

In the hydrolysis step, the yeast cell wall is hydrolyzed with, for example, an alkaline aqueous solution to obtain a hydrolysate. For example, an aqueous solution containing an alkali metal hydroxide such as sodium hydroxide is used as the alkaline aqueous solution. The concentration of the alkaline aqueous solution is, for example, 0.01 mass% or more and 10 mass% or less, preferably 0.1 mass% or more and 5 mass% or less, more preferably 1 mass% or more and 3 mass% or less. The temperature of the hydrolysis treatment is, for example, 70 °C or higher and 100 °C or lower, preferably 85 °C or higher and 95 °C or lower. The time of the hydrolysis treatment is, for example, 1 hour or more and 48 hours or less, preferably 4 hours or more and 24 hours or less.

The recovery step includes, for example, a neutralization step of neutralizing the hydrolysate to obtain a neutralized product if necessary, a second centrifugation step of centrifuging the neutralized product or the hydrolysate to obtain a glucan-containing composition, and a drying step of drying the glucan-containing composition. By performing an alkaline hydrolysis treatment of the yeast cell wall obtained from yeast autolysate etc., the immunostimulator containing glucan and lipid can be produced with stable quality.

In the neutralization step, an acidic compound is added to the alkaline hydrolysate to adjust pH from 6.5 to 7.5 to obtain a neutralized product. Inorganic acids such as hydrochloric acid, nitric acid, sulfuric acid, and phosphoric acid, or organic acids such as formic acid and acetic acid are used for the acidic compound. In the second centrifugation step, the neutralized product is centrifuged to obtain a glucan-containing composition as a heavy liquid. For centrifugation, for example, a nozzle centrifuge, an intermittent discharging centrifuge, a Sharples centrifuge, etc. can be used in industrial production, and conditions for centrifugation can appropriately be adjusted as long as an insoluble component in the neutralized product or hydrolysate can be recovered. In the drying step, the glucan-containing composition is dried to obtain the immunostimulator. Regarding drying conditions, a spray dryer, a freeze dryer, a drum dryer, etc. can be used in industrial production, and the conditions can appropriately be adjusted. A sterilization step of sterilizing the glucan-containing composition may be included before the drying step. The sterilization step can be performed by performing a heat treatment at 120 °C or higher for 10 to 60 seconds with a UHT sterilizer, for example, and can appropriately be adjusted to satisfy a desired quality.

### Method for Manufacturing Yeast-Derived Glucan

A method for manufacturing yeast-derived glucan according to this embodiment includes a providing step of providing a composition containing an autolyzed yeast cell wall, a hydrolysis step of performing an alkaline hydrolysis treatment of the composition containing the yeast cell wall to obtain a hydrolysate, and a recovery step of recovering glucan from the hydrolysate. The yeast-derived glucan obtained by alkaline hydrolysis of the yeast cell wall obtained by autolyzing yeast cells exhibits favorable quality stability and suppresses quality variations among production lots. The manufactured yeast-derived glucan has a good recovery rate of glucan, and impurities such as mannan and protein are sufficiently removed. The method for manufacturing yeast-derived glucan may be a method for purifying yeast-derived glucan.

In the providing step, the composition containing an autolyzed yeast cell wall is provided. The composition containing the yeast cell wall may be appropriately selected and provided from commercially available products, or the composition containing the yeast cell wall having a desired composition may be manufactured and provided. The yeast used as a raw material is as described above.

The composition containing the yeast cell wall can be manufactured by, for example, a method including an autolysis step of autolyzing the yeast cells used as a raw material to obtain an autolysate, and a first centrifugation step of centrifuging the autolysate. Details of the autolysis step and the first centrifugation step are as described above.

In the method for obtaining the composition containing the yeast cell wall, yeast cells used as a raw material may be subjected to a purification treatment before the autolysis step. The purification treatment includes, for example, removing contaminants by sieving the yeast cells used as a raw material with a vibrating sieve, washing under alkaline conditions at pH 9 to 11, etc.

In the hydrolysis step, the yeast cell wall is hydrolyzed with an alkaline aqueous solution to obtain a hydrolysate. Details of the hydrolysis step are as described above.

In the recovery step, glucan is recovered from the hydrolysate. The glucan obtained in the recovery step is obtained as a glucan-containing composition, for example. Details of the recovery step are as described above.

The method for manufacturing yeast-derived glucan is excellent in removal rates of mannan, protein, etc. while achieving a good recovery rate of glucan. The recovery rate of glucan is calculated by dividing the total amount of glucan contained in the obtained yeast-derived glucan by the total amount of glucan contained in the composition containing the autolyzed yeast cell wall. The removal rate of mannan is calculated by dividing, by the total amount of mannan contained in the composition containing the autolyzed yeast cell wall, a removal amount obtained by subtracting the total amount of mannan contained in the obtained yeast-derived glucan from the total amount of mannan contained in the composition containing the autolyzed yeast cell wall. The same applies to the removal rates of protein etc.

The recovery rate of glucan in the method for manufacturing yeast-derived glucan is, for example, 65 % or more, preferably 70 % or more, or 75 % or more. An upper limit of the recovery rate of glucan is, for example, 95 % or less, preferably 90 % or less, or 85 % or less.

The removal rate of mannan in the method for manufacturing yeast-derived glucan is, for example, 85 % or more, preferably 90 % or more, or 95 % or more. An upper limit of the removal rate of mannan is, for example, 100 % or less, preferably less than 100 %.

The removal rate of protein in the method for manufacturing yeast-derived glucan is, for example, 80 % or more, preferably 85 % or more, or 90 % or more. An upper limit of the removal rate of protein is, for example, 100 % or less, preferably less than 100 %, or 98 % or less.

The present invention includes, as another aspect, an immunostimulation method including administrating an immunostimulator or yeast-derived glucan to a subject. The present invention includes, as other aspects, a use of an immunostimulator or yeast-derived glucan in manufacturing of a composition used in the immunostimulation method or the method for preventing infectious diseases, a use of an immunostimulator or yeast-derived glucan in the immunostimulation method or the method for preventing infectious diseases, and an immunostimulator or yeast-derived glucan used in the immunostimulation method or the method for preventing infectious diseases. Subjects of the immunostimulating method or the method for preventing infectious diseases may be the vertebrates described above and include mammals, birds, fish, etc.

### Examples

The present invention will hereinafter specifically be described with reference to Examples; however, the present invention is not limited to these Examples.

### Method for Manufacturing Immunostimulator 1

Yeast cell walls (15 % solid content; derived from yeast autolysate) derived from beer yeast belonging to the genus Saccharomyces manufactured at Tochigi Koganei Plant of Asahi Group Foods Co., Ltd. were prepared and subjected to a hydrolysis treatment. Specifically, sodium hydroxide was added to a slurry containing the yeast cell walls to a final concentration of about 1.5 mass% or about 2.0 mass%, and the slurry was heated to 90 °C for the hydrolysis treatment for 4 hours. The treated yeast cell wall slurry was adjusted to pH 7.0 with hydrochloric acid and was then subjected to batch centrifugation at 8,000 g×10 minutes by using a high-speed cooling centrifuge (Avanti J-26S XP manufactured by BECKMAN COULTER). After a precipitate was suspended by adding 1.5 L of distilled water, centrifugation was performed under the conditions described above. This operation was repeated four times, and the precipitate was dried in a freeze dryer (FDU-2100 manufactured by EYELA) for two nights or more to obtain an immunostimulator.

### Method of Manufacturing Immunostimulator 2

Yeast cell walls (15 % solid content; derived from yeast autolysate) derived from beer yeast belonging to the genus Saccharomyces manufactured at Tochigi Koganei Plant of Asahi Group Foods Co., Ltd. were prepared and subjected to a hydrolysis treatment. Specifically, sodium hydroxide was added to a slurry containing the yeast cell walls to a final concentration of about 1.5 mass% or about 2.0 mass%, and the slurry was heated to 90 °C for the hydrolysis treatment for 4 hours. After the treated yeast cell wall slurry was adjusted to pH 7.0 with hydrochloric acid and was then separated to solid and liquid while adding water by using nozzle-type continuous centrifuges (FEUX512T-31C-50 and FEUX412U-31C-50 manufactured by Alfa Laval), and obtained heavy liquid was sterilized under the conditions of 130 °C for 40 seconds and then dried in a drum dryer to obtain an immunostimulator.

### Glucan Analysis Method 1

After approximately 0.6 g of a sample was weighed and 4 mL of 72 (w/w)% sulfuric acid was added, the mixture was stirred at room temperature for 1 hour. Subsequently, the sulfuric acid concentration was adjusted to 4 (w/w)% with Milli-Q (registered trademark) water, and a reaction was performed at 121 °C for 1 hour. After cooling, the mixture was neutralized with an aqueous sodium hydroxide solution. After adjustment to constant volume, filtration was performed, and the filtrate was subjected to HPLC. The column was Wakopak (registered trademark) Wakosil (registered trademark) 5NH₂ (ø4.6 mm×250 mm, FUJIFILM Wako Pure Chemical Corporation), the column temperature was room temperature, the mobile phase was acetonitrile:water=75:25, the flow rate was 1.0 mL/min, and the injection amount was 2 µL. For a reaction solution, a mixed solution of 1 (w/v)% arginine and 3 (w/v)% boric acid was added at 0.7 mL/min, and reacted at 150 °C to detect glucose by using a fluorescence detector (excitation wavelength: 320 nm, measurement wavelength: 430 nm). An obtained glucose content percentage was multiplied by 0.9 to calculate a total content percentage of glucose.

### Glucan Analysis Method 2

Approximately 0.5 g of a sample was weighed, 25 ml of 0.08 mol/l phosphate buffer (pH 6.0) was added, 0.1 ml of Termamyl 120L (Novozymes) was added, and the mixture was reacted in a boiling water bath for 30 minutes. After radiational cooling, pH was adjusted to 7.5±0.1 with a 0.275 mol/l sodium hydroxide solution, and protease (Sigma-Aldrich, P-5380) was allowed to act at 60 °C for 30 minutes. After radiational cooling, pH was adjusted to 4.3±0.1 with 0.325 mol/l hydrochloric acid, and amyloglucosidase (Sigma-Aldrich, A-9913) was allowed to act at 60 °C for 30 minutes. To the liquid after the enzyme treatment, four times the amount of 95 % ethanol was added, and the mixture was allowed to stand for 1 hour or more. The liquid was poured into a filter containing diatomaceous earth, and suction filtration was performed. The residue was washed with ethanol and acetone. The residue was recovered, 5 ml of 72 (w/w)% sulfuric acid was added, and the mixture was decomposed at 20 °C for 4 hours. Water was added to a concentration of 4 (w/w)%, and the mixture was decomposed in a boiling water bath for 2 hours. Radiational cooling was followed by neutralization, adjustment to constant volume, and filtration. Glucose in the filtrate was quantified by a glucose oxidase method, and a glucose concentration was obtained and multiplied by 0.9 to calculate the content percentage of β-glucan.

The content percentage of α-glucan was calculated by subtracting the content percentage of β-glucan from the total content percentage of glucan obtained in Analysis Method 1.

### Lipid Analysis Method

Quantification was performed by an acid decomposition method. To a collected sample, 2 ml of ethanol and 10 ml of concentrated hydrochloric acid were added, and the sample was decomposed in a constant temperature bath at 70 °C to 80 °C for 30 to 40 minutes. Subsequently, the sample was transferred to a Mojonnier tube, mixed with ethanol and diethyl ether, and further mixed with petroleum ether. An ether layer was recovered, and a liquid mixture of diethyl ether and petroleum ether was further added to an aqueous layer for partitioning. The ether layer was recovered, and the aqueous layer was partitioned again by using diethyl ether and petroleum ether to recover the ether layer. Diethyl ether and petroleum ether were distilled away and then dried at 105 °C for 1 hour, and a weight difference before and after drying was obtained as a lipid amount to calculate a lipid content percentage from a sample collection amount.

### Mannan analysis method

After approximately 0.6 g of a sample was weighed and 4 ml of 72 (w/w)% sulfuric acid was added, the mixture was stirred at room temperature for 1 hour. Subsequently, Milli-Q (registered trademark) water was added to achieve the sulfuric acid concentration of 4 (w/w)%, and hydrolysis was performed at 121 °C for 1 hour. After radiational cooling, the mixture was neutralized and adjusted to constant volume, and a content of mannose in a filtrate was quantified in the same manner as the glucan analysis method described above. The mannose concentration in the sample was calculated and multiplied by 0.9 to calculate a mannose content percentage.

### Protein Analysis Method

A protein content percentage in a sample was quantified by a combustion method. A total nitrogen measuring device (Sumika Chemical Analysis Service) was used for the analysis. The sample was collected in a quartz boat, a reaction furnace temperature was set to 870 °C or higher, and the analysis was performed by setting a reduction furnace temperature to 600 °C, a detector temperature to 100 °C, and a column temperature of 70 °C. A detected nitrogen gas was quantified and multiplied by a nitrogen-protein conversion coefficient of 6.25 to calculate a protein content percentage.

### Ash Analysis Method

An ash content percentage in a sample was quantified by a direct ashing method. A sample was collected in a porcelain crucible and pre-ashing was performed. Subsequently, ashing was performed at 550 °C. After radiational cooling in a silica gel desiccator, the weight was measured. A weight difference before and after ashing was obtained as an ash amount to calculate an ash content percentage from a sample collection amount.

Table 1 shows results of component analysis of 14 samples of the immunostimulator obtained by the manufacturing method described above. The content percentages of the components shown in Table 1 are dry matter-equivalent values of the immunostimulator calculated on a mass basis. The content percentage of glucan in Table 1 is the total content percentage of α-glucan and β-glucan, and the same applies hereinafter.

**[Table 1]**

| | NaOH 1.5% | | | | | | NaOH 2.0% | | | | | | | | All samples | | |
|---|---|---|---|---|---|---|---|---|---|---|---|---|---|---|---|---|---|
| | Manufacturing method 1 | | | | Manufacturing method 2 | | Manufacturing method 1 | | | | | Manufacturing method2 | | | Average | Standard deviation | Variant coefficient (%) |
| | #1 | #2 | #3 | #4 | #5 | #6 | #7 | #8 #9 | | #10 | #11 | #12 | #13 | #14 | | | |
| protein (%) | 5.1 | 5.1 | 5.4 | 5.1 | 6.2 | 6.3 | 4.0 | 3.8 | 3.0 | 4.1 | 4.1 | 6.9 | 5.9 | 4.3 | 5.0 | 1.11 | 22.5 |
| lipid (%) | 15.1 | 13.8 | 13.9 | 12.7 | 13.9 | 14.2 | 12.9 | 14.0 | 9.1 | 10.7 | 13.2 | 10.2 | 12.8 | 12.2 | 12.8 | 1.69 | 13.2 |
| ash (%) | 2.1 | 1.9 | 2.0 | 2.1 | 2.5 | 2. 6 | 2.5 | 2.9 | 1.2 | 2.6 | 2.4 | 5.1 | 4.8 | 3.5 | 2.7 | 1.07 | 39.1 |
| carbohydrate (%) | 77.6 | 79.2 | 78.7 | 80.0 | 77.4 | 76.9 | 80.6 | 79.2 | 86. 6 | 82.5 | 80.3 | 77.8 | 76.5 | 80.0 | 79. 5 | 2.64 | 3.3 |
| *β*-glucan (%) | 47.1 | 48.0 | 49.4 | 48.4 | 41.2 | 44.5 | 53.8 | 56.2 | 45.4 | 41.1 | 51.2 | 43.0 | 45. 2 | 48.9 | 47.4 | 4.42 | 9.3 |
| *α*-glucan (%) | 24. 6 | 24.0 | 20.1 | 20.2 | 30.3 | 25.2 | 19.9 | 14.4 | 32.7 | 32. 7 | 20.2 | 24.1 | 23. 1 | 22.7 | 23.9 | 5.18 | 21.7 |
| glucan (%) | 71.7 | 72.1 | 69.5 | 68.6 | 71.5 | 69. 7 | 73.6 | 70.6 | 78.2 | 73.7 | 71.3 | 67.1 | 68.3 | 71.5 | 71.2 | 2.77 | 3.9 |
| mannan (%) | 0.7 | 0.7 | 0.6 | 0.6 | 1.1 | 1.1 | 0.6 | 0.5 | 0.5 | 0.6 | 0.7 | 2.2 | 1.4 | 1.1 | 0.9 | 0.47 | 51.7 |
| lipid + glucan (%) | 86.9 | 85.9 | 83.4 | 81.3 | 85.4 | 83.9 | 86.5 | 84.6 | 87.3 | 84.4 | 84.5 | 77.3 | 81.1 | 83.8 | 84.0 | 2.66 | 3.2 |
| *α*-glucan/ *β*-glucan | 0.52 | 0.50 | 0.41 | 0.42 | 0.74 | 0.57 | 0.37 | 0. 26 | 0.72 | 0. 80 | 0.39 | 0.56 | 0.51 | 0.46 | 0.52 | 0.15 | 29.5 |
| lipid/glucan | 0.21 | 0.19 | 0.20 | 0.19 | 0.19 | 0.20 | 0.17 | 0.20 | 0.12 | 0.15 | 0.18 | 0.15 | 0.19 | 0.17 | 0.18 | 0.03 | 14.5 |

As a reference example, 9 samples of a commercially available immunostimulator containing β-1,3/1,6-glucan as a main component (hereinafter, also referred to as "commercial product A") were obtained from different production lots, and the component analysis was performed in the same manner. Table 2 shows average values, standard deviations, and coefficients of variation (%) of content percentages of the components. The content percentages of the components shown in Table 2 are dry matter-equivalent values on a mass basis.

**[Table 2]**

| | Average | Standard deviation | Variant coefficient (%) |
|---|---|---|---|
| protein (%) | 4.7 | 1.68 | 35.4 |
| lipid (%) | 17.8 | 4.98 | 28.0 |
| ash (%) | 3.7 | 2.74 | 74.3 |
| carbohydrate (%) | 73.8 | 7.26 | 9.8 |
| *β*-glucan (%) | 52.5 | 6.09 | 11.6 |
| *α*-glucan (%) | 8.6 | 3.30 | 38.6 |
| total glucan (%) | 61.0 | 8.49 | 13.9 |
| mannan (%) | 1.4 | 0.72 | 50.8 |
| lipid + glucan (%) | 78.8 | 5. 53 | 7.0 |
| *α*-glucan/ *β*-glucan | 0.16 | 0.05 | 31.3 |
| lipid/glucan | 0.30 | 0.10 | 33.7 |

As shown in Table 1, the average value of the total content percentage of lipid and glucan of the immunostimulator was 80 mass% or more, and the variation in the content percentage among the samples was small. In contrast, as shown in Table 2, the average value of the total content percentage of lipid and glucan of the commercial product A was less than 80 mass%, and the variation among the samples was large.

The immunostimulator obtained as described above and the commercial product A were evaluated for an ability to promote production of reactive oxygen species (ROS) for leukocytes.

### Ability to Promote Production of ROS for Porcine Leukocytes

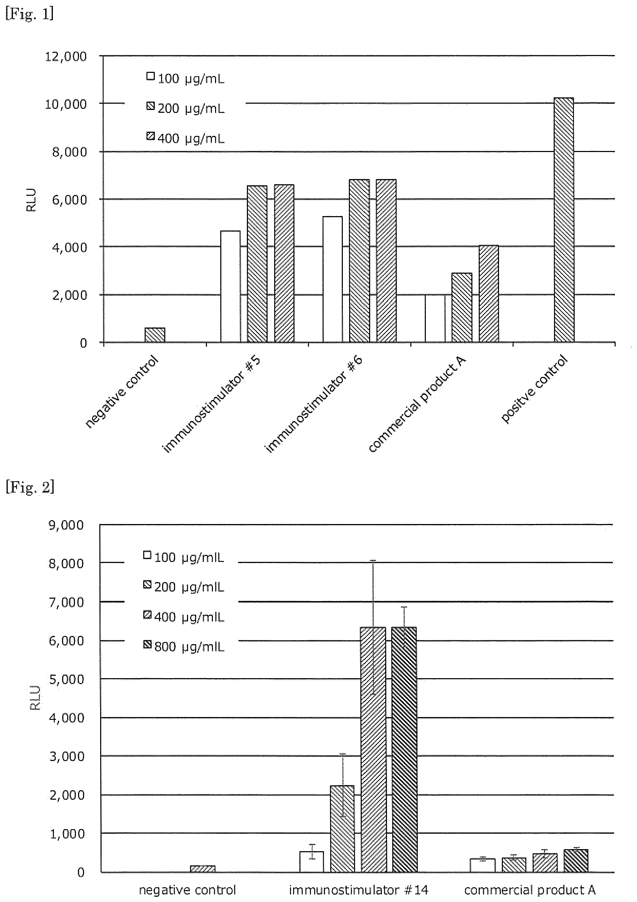

Monocytes were isolated from peripheral blood collected from a 10-week-old weaned piglet. Specifically, peripheral blood mononuclear cells (PBMC) suspended in RPMI1640 medium containing 10 % fetal bovine serum were seeded on a 96-well plate at 2×10⁶ cells/well and then cultured for 2 hours for adhesion and fixation of the monocytes in the wells. After removing the culture supernatant and washing with HBSS (Hanks' Balanced Salt Solution) to remove lymphocytes, the immunostimulator (#5, #6) or the commercial product A was added to each well together with a luminescent reagent (luminol) and HBSS to achieve a final concentration from 100 µg/mL to 400 µg/mL. Subsequently, an integrated amount of luminescence for 120 minutes was measured with a luminometer (Thermo Fisher Scientific). To a negative control, only the luminescent reagent (luminol) and HBSS were added, and a relative luminescent unit (RLU) was evaluated as a ROS production amount. To a positive control, phorbol 12-myristate 13-acetate (PMA) was added at 50 µg/mL together with the luminescent reagent (luminol) and HBSS. The results are shown in Fig. 1.

As shown in Fig. 1, the immunostimulator #5, #6 of two different production lots has a ROS production amount greater than the commercial product A at all the addition concentrations of 100 µg/mL to 400 µg/mL. The total content percentage of glucan and lipid of the immunostimulator #5 was 85.4 mass%, and the total content percentage of glucan and lipid of the immunostimulator #6 was 83.9 mass%.

### Ability to Promote Production of ROS for Human Leukocytes

The human leukemia cell line HL-60 was cultured in RPMI1640 medium containing 1.25 vol% dimethyl sulfoxide and 10 % fetal bovine serum for 6 days to induce neutrophil-like differentiation in accordance with the method of Collins et al. (Collins SJ, Ruscetti FW, Gallagher RE, Gallo RC, Proc. Natl. Acad. Sci. USA, 75, 2458-2462, 1978). The neutrophil-like HL-60 suspended in the medium described above was seeded on a 96-well plate at 4×10⁵ cells/well and then adhered and fixed in the wells by culturing for 1 hour for adhesion and fixation in the wells, the immunostimulator #14 or the commercial product A was added to each well together with a luminescent reagent (luminol, manufactured by Nacalai Tesque) and HBSS to achieve a final concentration of 100 µg/mL to 800 µg/mL. Subsequently, an integrated amount of luminescence for 120 minutes was measured with a luminometer. To a negative control, only the luminescent reagent (luminol) and HBSS were added, and a relative luminescent unit (RLU) was evaluated as a ROS production amount. The results are shown in Fig. 2.

As shown in Fig. 2, the immunostimulator #14 of the present invention has a larger ROS production amount than the commercial product A at all the addition concentrations of 100 µg/mL to 800 µg/mL. The total content percentage of glucan and lipid of the immunostimulator #14 used in this case was 83.8 mass%.

### Rainbow Trout Vibrio Disease Infection Test

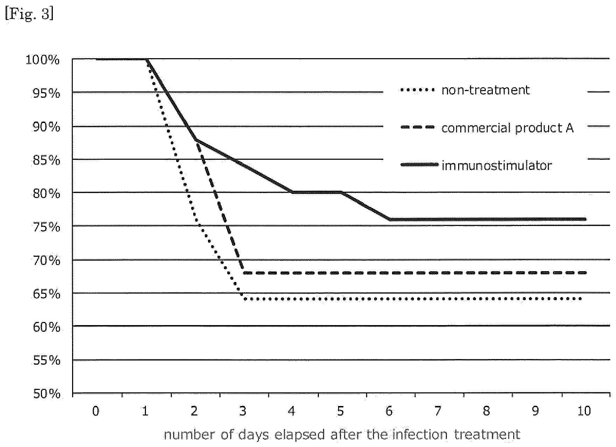

Twenty-five rainbow trouts having an average body weight of 2 g were reared with a feed having the immunostimulator #4 or the commercial product A spread at the final concentration of 0.2 mass%. After seven days from the start of the raising, 0.05 mL of Vibrio anguillarum diluted with PBS to 9×10⁴ cfu/mL was intraperitoneally injected to the rainbow trouts for infection treatment. Subsequently, the raising was continued for 10 days, and a survival rate was calculated every day. The results are shown in Fig. 3. In Fig. 3, the vertical axis indicates the survival rate (%), and the horizontal axis indicates the number of days elapsed after the infection treatment.

The survival rate on the 7th day after injection was 64 % for the negative control (without treatment), 76 % for the immunostimulator administration group, and 68 % for the commercial product A administration group. The total content percentage of glucan and lipid of the immunostimulator #4 used in this case was 81.3 mass%.

As shown in Fig. 3, the immunostimulator of the present invention showed a higher survival rate than the commercially available immunostimulator.

The disclosures of Japanese Patent Application No. 2018-191154 (Filing Date: October 9, 2018) is hereby incorporated by reference in its entirety. All the documents, patent applications, and technical standards described in this description are hereby incorporated by reference to the same extent as if each of the documents, patent applications, and technical standards is specifically and individually described as being incorporated by reference.

## Claims

1. An immunostimulator comprising: glucan and lipid derived from a yeast cell wall, the immunostimulator having a total content percentage of glucan and lipid of 80 mass% or more, having a content ratio of lipid to glucan of 0.1 or more and 0.4 or less, and being water-insoluble.

2. The immunostimulator according to claim 1, wherein a content ratio of α-glucan to β-glucan is 0.2 or more and 0.85 or less.

3. The immunostimulator according to claim 1 or 2, comprising mannan, wherein a content percentage of mannan is 5 mass% or less.

4. The immunostimulator according to any one of claims 1 to 3, wherein the immunostimulator is used for fish.

5. A food/drink or a feed comprising: the immunostimulator according to any one of claims 1 to 4.

6. An infection preventive agent for fish comprising: glucan and lipid, the infection preventive agent having a total content percentage of glucan and lipid of 80 mass% or more.

7. A method for preventing infection comprising: administering to a subject the immunostimulator according to any one of claims 1 to 4.

8. A method for manufacturing yeast-derived glucan, comprising:
providing a composition containing a yeast cell wall subjected to an autolysis treatment;
subjecting the composition containing the yeast cell wall to an alkaline hydrolysis treatment to obtain a hydrolysate; and
recovering glucan from the hydrolysate.

9. The method for manufacturing according to claim 8, wherein
the composition containing the yeast cell wall is obtained by a method including
subjecting a yeast to an autolysis treatment to obtain an autolysate, and
subjecting the autolysate to a centrifuging treatment.

10. The method for manufacturing according to claim 8 or 9, wherein the alkali hydrolysis treatment is performed by heating in the presence of an alkali metal hydroxide.

11. The method for manufacturing according to any one of claims 8 to 10, wherein the yeast-derived glucan has a content percentage of mannan of 5 mass% or less.

12. The method for manufacturing according to any one of claims 8 to 11, wherein the yeast-derived glucan has a content percentage of protein of 10 mass% or less.

13. The method for manufacturing according to any one of claims 8 to 12, wherein the yeast-derived glucan has a coefficient of variation of glucan content percentage of 10 % or less.
